# FASCICULE DE BREVET EUROPEEN

(11) **EP 3 212 615 B1**
(45) Date de publication et mention de la délivrance du brevet: **29.08.2018**
(21) Numéro de dépôt: 15797123.5
(22) Date de dépôt: 19.10.2015
(51) Int. Cl.: C07D 207/16, A61K 8/42, A61K 8/49, A61Q 19/08, C07C 233/05

(54) **UTILISATION D'ESTER DE DÉRIVÉS N-ACYLÉS D'ACIDE AMINÉS ET DE POLYOLS COMME AGENT ANTIVIEILLISSEMENT DE LA PEAU HUMAINE**
VERWENDUNG VON ESTERN DES N-ACYLIERTEN DERIVATEN VON AMINOSÄUREN UND POLYOLEN ALS ANTIALTERUNGSMITTEL FÜR MENSCHLICHE HAUT
USE OF ESTERS OF N-ACYLATED DERIVATIVES OF AMINO ACIDS AND POLYOLS AS AN ANTI-AGEING AGENT FOR HUMAN SKIN

(30) Priorité: 27.10.2014 FR 1460294
(43) Date de publication de la demande: 06.09.2017
(73) Titulaire: Société d'Exploitation de Produits pour les Industries Chimiques SEPPIC, 75321 Paris Cedex 7 (FR)
(72) Inventeur: CATTUZZATO, Laetitia, F-81100 Castres (FR); DUMONT, Sandy, F-81200 Caucalieres (FR); GUILBOT, Jerôme, F-81100 Castres (FR)
(74) Mandataire: Conan, Philippe Claude
(86) Numéro de dépôt international: PCT/FR2015/052799
(87) Numéro de publication internationale: WO 2016/066923

(56) Documents cités:
- WO-A1-2013/001192
- FR-A1- 2 936 152
- US-A- 4 240 823
- US-A- 5 266 576

## Description

La présente invention a pour objet l'utilisation d'esters de dérivés N-acylés d'acides aminés et de polyols comme agent antivieillissement de la peau du corps humain, ainsi que les compositions cosmétiques, pharmaceutiques, dermo-pharmaceutique à usage topique comprenant lesdits esters de dérivés N-acylés d'acides aminés et de polyols destinées à prévenir le vieillissement la peau du corps humain.

La peau humaine constitue la première image offerte au regard d'autrui, et par conséquent, l'amélioration de son aspect est un sujet de préoccupation constant pour les êtres humains. La peau est le reflet d'un état de bien-être, souvent associé à la jeunesse, et a *contrario* à un état de fatigue et/ou de vieillissement. Le vieillissement cutané est donc une préoccupation pour les humains et plus particulièrement pour les consommateurs de produits cosmétiques qui recherchent des solutions pour atténuer et/ou pour prévenir les manifestations visibles liées dudit vieillissement. Ce vieillissement cutané s'observe au niveau des différents tissus cutanés et se caractérise par des altérations métaboliques, fonctionnelles, cellulaires, architecturales et tissulaires, conduisant à des effets externes visibles caractérisés par l'apparition et l'accroissement des rides, par un teint terne, par un manque d'uniformité du teint (phénomène de dyschromie), ou encore par une modification de la texture et des propriétés, notamment biomécaniques, de la peau du corps humain.

Le vieillissement cutané résulte d'une part de facteurs propres à chaque individu (caractéristiques du patrimoine génétique propre à chaque individu) et d'autre part de facteurs environnementaux. Parmi les facteurs environnementaux qui peuvent provoquer le vieillissement cutané, on peut citer l'exposition répétée et prolongée au soleil, et plus particulièrement l'exposition aux rayonnements ultra-violets, l'exposition à la pollution atmosphérique, à la fumée de cigarette, les stress oxydants divers pouvant résulter entre autres des facteurs précédemment cités, ainsi que les stress psychologiques, émotionnels et nerveux. L'exposition répétée et prolongée de la peau humaine aux rayonnements du soleil, et plus particulièrement aux rayonnements ultra-violets, conduit à une forme de vieillissement que l'on nomme communément le photo-vieillissement. Ce photo-vieillissement est bien documenté dans la littérature scientifique et il provoque des altérations de la peau à différents niveaux dont une des altérations de la peau la plus connue est l'élastose solaire, qui se caractérisé par des modifications profondes dans l'architecture et l'organisation des fibres élastiques du derme. Ces modifications conduisent à un aspect caractéristique de ces peaux qui présentent des rides très profondes et marquées, induisant un aspect de peau tannée, à savoir raide, craquelée et brunie, ainsi que des modifications de leurs propriétés mécaniques. Les modifications des propriétés mécaniques de la peau humaine, liées au vieillissement, sont dues à l'altération de la matrice extracellulaire dermique, composée par les fibres élastiques et les fibres de collagène, et également à l'altération des caractéristiques cellulaires. *Schulze et al.*(1) ont montré que les fibroblastes dermiques se rigidifiaient avec l'âge, influençant des fonctions cellulaires impliquant le cytosquelette, telles que les propriétés contractiles, migratoires et prolifératives, qui sont importantes pour la réorganisation de la matrice extracellulaire.

Il est également connu que les espèces réactives de l'oxygène (connues sous le nom de « ROS ») en excès dans la peau humaine (que le stimulus soit exogène ou que la production soit endogène) créent des liaisons irréversibles avec les protéines, identifiées sous le terme « protéines carbonylées », qui perdent alors leur fonction. Un lien a récemment été mis en évidence entre ces protéines carbonylées et leur impact sur des fonctions clés cellulaires telles que le métabolisme des carbohydrates, l'entretien des protéines, la mobilité cellulaire, incluant la migration, et l'homéostasie protéique (2). *(Baraibar et Friguet, 2013).* Des études investiguant l'effet de sérums humains, provenant de donneurs de différents âges, sur les propriétés migratoires des fibroblastes ont été conduites par *Kondo et al.* (3). Les données obtenues montrent que le sérum de donneurs âgés inhibe les propriétés migratoires des fibroblastes, et même celles de fibroblastes foetaux. Ceci illustre l'importance des facteurs intrinsèques dans la problématique du vieillissement cutané.

La technique dite «photodynamique » a été décrite comme étant particulièrement adaptée pour le rajeunissement (i.e. la réduction des rides et ridules, des tâches pigmentaires, ...) des peaux dites « photo-exposées », à savoir les peaux exposées aux rayonnements solaires, et plus particulièrement aux rayonnements ultra-violets. Le mécanisme par lequel cette technique agit a récemment été étudié (4), et il s'avère que son mode d'action passe notamment par une augmentation de la population fibroblastique ainsi que par une augmentation de la capacité migratoire desdits fibroblastes.

Ces récentes études montrent donc que l'altération des propriétés migratoires des fibroblastes contribue au phénomène de vieillissement cutané. Ces propriétés migratoires sont importantes et décrites dans le cadre du processus de réparation des lésions cutanées. Des dysfonctionnements de ce processus chez les personnes âgées sont largement décrits, illustrant donc l'importance de cette fonctionnalité cellulaire.

Il en résulte qu'une amélioration des propriétés migratoires des fibroblastes du derme de la peau humaine et/ou qu'une augmentation de la population fibroblastique constituent des moyens de prévenir et/ou traiter le vieillissement de le peau du corps humain, et plus particulièrement de prévenir et/ou de traiter les effets visibles dudit vieillissement par exemple les rides, le teint terne, le manque d'uniformité du teint (dyschromie), la rigidité de la peau du corps humain, causé par le vieillissement naturel ou par une exposition prolongée au soleil, et plus particulièrement à une exposition aux rayonnements ultra-violets, ou par une exposition à des stresses oxydants.

Des procédés physiques destinés à stimulés la migration des fibroblastes de la peau humaine sont connus, et parmi ceux-ci, on peut citer l'irradiation au laser de faible intensité (exposition à une longueur d'onde de 632,2 nm) plus particulièrement destinée aux patients atteints diabète (5). Il existe également de nombreux principes actifs pharmaceutiques qui stimulent la migration des fibroblastes, et qui sont principalement prescrits dans le cadre de processus de cicatrisation. On peut ainsi citer la lactoferrine humaine recombinante produite par des plans de riz transgéniques (6). Ces procédés physiques et ces principes actifs pharmaceutiques sont généralement mis en oeuvre pour des individus atteints de pathologie, et ne sont pas adaptés pour des utilisations cosmétiques.

Il existe également des principes actifs cosmétiques qui sont des extraits de plantes, de bactéries, et qui sont décrits comme agissant sur la migration des fibroblastes. On peut ainsi citer les extraits de curcumin, qui stimulent la cicatrisation lorsqu'utilisés à faibles doses et agissent sur la migration des fibroblastes à plus forte dose (7) ; les huiles de noix de Pouteria Lucuma, se caractérisant par la présence majoritaire d'acide linoléique, d'acide oléique, d'acide palmitique, d'acide stéarique et d'acide γ linolénique, qui sont décrites comme stimulant la migration des fibroblastes et de l'expression de la vinculine, et adaptées à l'accélération de la cicatrisation des plaies de la peau (8) ; la combinaison d'extraits de Vigna Marina, Cocos Nucifera L., Terminalia Catappa L. et Hibiscus tiliaceus L. présente dans des compositions cosmétiques pour permettre de traiter les plaies, d'améliorer la cicatrisation, de traiter les problèmes cutanés liés à l'âge, décrite dans la demande internationale publiée sous le numéro WO 2010/127396 A1. La demande internationale publiée sous le numéro WO 2010 /056908 A1 divulgue l'utilisation d'extrait de Pouteria lucuma et plus particulièrement des huiles contenues dans leur noyau, pour améliorer la migration des fibroblastes humains. L'utilisation d'extraits de plantes, de bactéries, présente comme inconvénient de montrer des performances non fiables dans le temps du fait de la variabilité du contenu des matières premières.

Il existe également des principes actifs cosmétiques de synthèse par exemple des peptides qui sont décrits comme agissant sur la migration des fibroblastes. On peut ainsi citer des pentapeptides de formule Lys-Thr-Thr-Lys-X où X représente quelconque acide aminé naturel mais préférentiellement la sérine et dont une chaîne d'acides gras (C2 à C22) est greffée sur l'amine N-terminale et/ou son groupe carboxyle estérifié, décrits dans la demande de brevet français publiée sous le numéro FR 2 783 169. Ces pentapeptides sont incorporés dans des compositions cosmétiques ou pharmaceutiques pour provoquer l'augmentation de la synthèse de collagène et de glycosaminoglycanes (par radioactivité) sur des explants cutanés et l'augmentation de la prolifération de fibroblastes humains normaux en culture, et par conséquent pour améliorer l'aspect de la peau dans le cas de son vieillissement naturel ; de son dessèchement, de sa cicatrisation. La demande internationale publiée sous le numéro WO 97/17835 divulgue des peptides contenant au moins une séquence de trois acides aminés (Lys-Lys-Gly, Gly-His-Lys ou Glu-His-Lys) conjugués à un acide mono ou dicarboxylique, incorporés dans des compositions cosmétiques ou pharmaceutiques comme agent cicatrisant et antirides, montrant un effet sur la synthèse de collagène l par les fibroblastes.

Les dérivés N-acylés d'acides aminés sont des ingrédients chimiques largement employés pour la préparation de compositions cosmétiques, dermo-cosmétique, dermo-pharmaceutique et pharmaceutiques, en raison de leurs différentes propriétés actives.

On peut citer notamment la description de dérivés N-acylés lipophiles d'acides amines dans le brevet américain publié sous le numéro US 6,864,250, utilisés dans des méthodes de traitement de plaies et de brûlures. La demande de brevet européen publiée sous le numéro EP 505 868 A1 décrit des dérivés N-acylés d'acides aminés et leurs sels, ainsi que leur utilisation pour le traitement de l'ostéoporose et pour favoriser la cicatrisation. La demande de brevet hongrois publiée sous le numéro HU57582 décrit des dérivés N-acylés d'acides aminés et leurs sels, et plus particulièrement des dérivés N-acylés de la cystéine, ainsi que leur utilisation locale sur des plaies après application de lidocaïne. La demande de brevet japonais publiée sous le numéro JP 2002-179518 décrit des dérivés N-acylés de l'acide glutamique et de l'acide aspartique et leurs sels, se caractérisant par des chaînes acyles comportant de 6 à 24 atomes de carbone, utilisés pour être incorporés dans la préparation de compositions cosmétiques destinés aux peaux sensibles, sèches et atopiques.

La demande de brevet européen publiée sous le numéro EP 1 471 881 divulgue que des dérivés N-acylés d'acides α-aminés et notamment le N-undécylénoyl phénylalanine, présentent une affinité vis à vis du récepteur de la Mélanocyte Spécifique Hormone (α-MSH) et induisent ainsi l'éclaircissement de la peau selon le mécanisme biochimique suivant : la compétition entre l'hormone α-MSH et la molécule présentant une affinité vis à vis du récepteur α-MSH, entraîne un moindre taux de fixation de ladite hormone sur les récepteurs cellulaires ; cette compétition a pour conséquence une inhibition de l'activité de l'adénylate cyclase qui entraîne une moindre transformation de l'ATP en AMP cyclique intracellulaire ; la diminution du taux d'AMP cyclique entraîne une inhibition de l'enzyme Protéine Kinase A (PKA) ; l'inhibition de la Protéine Kinase A induit une moindre activation de la tyrosinase du fait de la moindre transformation de cette dernière en tyrosinase phosphorylée ; cette moindre activation de la tyrosinase entraîne la diminution de la synthèse de mélanine d'où découle une moindre pigmentation de la peau.

La demande de brevet japonais N° 2000-229121 décrit l'utilisation des polyols esters des N-acylamino acides comme tensioactifs performants.

La demande internationale publiée sous le numéro WO 2010/034917 décrit les mono-esters et les di-esters de polyols du N-(ω-undecylenoyl) phenylalanine, et plus particulièrement les mono-esters et les di-esters résultant de la réaction du glycérol et du N-(w-undecylenoyl) phenylalanine, et leurs utilisations comme agents éclaircissants de la peau humaine.

La demande internationale de brevet publiée sous le numéro WO 2013/001192 A1 décrit des mono-esters et des di-esters résultant de la réaction d'estérification entre de l'isosorbide et des dérivés N-acylés d'acides aminés, leurs utilisations comme agent actif cosmétique, pour prévenir et/ou limiter les effets inesthétiques générés par l'hypoxie des cellules endothéliales du corps humain, et plus particulièrement pour prévenir et/ou limiter les effets inesthétiques générés par les cernes, les poches péri-oculaires et/ou les jambes lourdes.

Dans le cadre de leurs recherches de nouveaux actifs cosmétiques pour la prévention et/ou le traitement des signes du vieillissement de la peau humaine ou des lèvres, les inventeurs se sont attachés à développer une nouvelle solution technique consistant en l'utilisation de produits résultant de la réaction d'estérification entre des polyols et des dérivés N-acylés d'acides aminés.

C'est pourquoi, selon un premier aspect, l'invention a pour objet l'utilisation non thérapeutique d'un composé de formule (I') :

R'-O-CH₂-CH(OH)-[CH₂-O-CH₂]ₛ-[CH(OH)]ₚ-CH₂-O-R" (I'),

formule (I') dans laquelle s est égal à 0 ou à 1, et p est égal à 0, 1, 2 ou 3, R' et R", identiques ou différents, représentent soit un atome d'hydrogène, soit un radical monovalent de formule (II'a) : 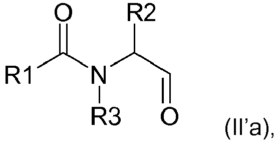 formule (II'a) dans laquelle le groupe R1-C(=O)- représente un radical choisi parmi les radicaux octanoyle, décanoyle, ω-undécylènoyle, dodécanoyle, tétradécanoyle, hexadécanoyle, octadécanoyle, cocoyle, eicosanoyle, 9-octadécènoyle, éicosènoyle, 9,12-octadécadiènoyle ou 9,12,15-octadécatriénoyle, R2 représente un atome d'hydrogène ou un radical choisi parmi les radicaux méthyle, isopropyle, isobutyle, 1-méthyl propyle, hydroxyméthyle, 1-hydroxy éthyle, 4-aminobutyle, 3-guanidino propyle, 3-uréido propyle, (1-amino carbonyl) méthyle, carboxy méthyle, 2-carboxy éthyle, 2-(amino carbonyl) éthyle, benzyle, 4-hydroxy benzyle, 3,4-dihydroxy benzyle, [1H-indol-3-yl]méthyle, (1H-imidazol-4-yl) méthyle, 3-amino propyle, et R3 représente un atome d'hydrogène ou un radical méthyle ; soit un radical monovalent de formule (II'b) : 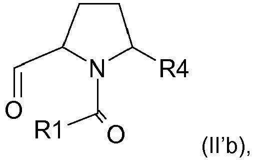 formule (II'b) dans laquelle le groupe R₁-C(=O)- représente un radical choisi parmi radicaux représente un radical choisi parmi les radicaux octanoyle, décanoyle, ω-undécylènoyle, dodécanoyle, tétradécanoyle, hexadécanoyle, octadécanoyle, cocoyle, eicosanoyle, 9-octadécènoyle, éicosènoyle, 9,12-octadécadiènoyle ou 9,12,15-octadécatriénoyle et R4 représente un atome d'hydrogène ou un radical hydroxy, étant entendu :
- qu'au moins un des radicaux R' ou R" ne représente pas un atome d'hydrogène,
- que lorsqu'aucun des radicaux R' et R" ne représente un atome d'hydrogène, R' et R" sont identiques mais ne représentent pas un radical monovalent de formule (II'a) dans laquelle le groupe R1-C(=O) représente le radical ω-undécylènoyle, R2 représente un radical benzyle et R3 représente un atome d'hydrogène et
- que lorsque l'un des radicaux R' et R" représente un atome d'hydrogène, l'autre ne représente pas un radical monovalent de formule (II'a) dans laquelle le groupe R1-C(=O) représente le radical ω-undécylènoyle, R2 représente un radical benzyle et R3 représente un atome d'hydrogène,
ladite utilisation étant dans le but d'empêcher ou de ralentir l'apparition des signes du vieillissement de la peau humaine ou des lèvres ou bien d'éliminer lesdits signes.

Par " signes du vieillissement de la peau humaine ou des lèvres" on entend, au sens de l'invention, toutes modifications de l'aspect extérieur de la peau ou des lèvres dues au vieillissement, qu'elles soient chronobiologiques et/ou photo-induites et/ou résultant de l'exposition à des stresses environnementaux (pollution atmosphérique, contact avec substances dangereuses), comme les rides et ridules, l'altération du microrelief, le manque d'élasticité et/ou de tonus de la peau, le manque de densité et/ou de fermeté de la peau humaine ou des lèvres, mais également toutes modifications internes de la peau qui ne se traduisent pas systématiquement par un aspect extérieur modifié, comme par exemple toutes dégradations internes de la peau consécutives à une exposition aux rayonnements ultra-violets.

Selon un premier aspect particulier l'invention a pour objet l'utilisation telle que définie ci-dessus, d'une composition (C'1) comprenant pour 100% de sa masse :
- De 99% massique à 20% massique d'au moins un composé de formule (I'a) :

   R'-O-CH₂-CH(OH)-[CH₂-O-CH₂]ₛ₋[CH(OH)]ₚ-CH₂-O-H (I'a),

   formule (I'a) correspondant à la formule (I') telle que définie précédemment dans laquelle R" représente un atome d'hydrogène,
- De 1% massique à 80% massique d'au moins un composé de formule (I'b) :

   R-O-CH₂-CH(OH)-[CH₂-O-CH₂]ₛ-[CH(OH)]ₚ-CH₂-O-R (I'b),
formule (Ib) correspondant à la formule (I') telle que définie précédemment dans laquelle R' et R" sont identiques et représentés par le radical R.

Selon un autre aspect particulier, l'invention a pour objet l'utilisation non thérapeutique d'un composé de formule (I') ou d'une composition (C'1) tels que définis précédemment, pour laquelle lesdits signes du vieillissement de la peau humaine ou des lèvres sont le manque d'élasticité et/ou de tonus de la peau humaine ou des lèvres, ou le manque de densité et/ou de fermeté de la peau humaine ou des lèvres, ou une altération du microrelief de la peau humaine ou des lèvres.

Selon un autre aspect particulier, les composé de formule (I'), formule (l'a) et de formule (I'b) mis en oeuvre dans l'utilisation objet de la présente invention, sont choisis choisi parmi les esters de polyols des dérivés N-acylés des acides aminés suivants : la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, l'hydroxyproline, l'hydroxylysine, la sarcosine ou l'ornithine.

Selon un autre aspect particulier de la présente invention, dans la formule (II'a), R2 représente un radical choisi parmi les radicaux méthyle, isopropyle, isobutyle, 1-méthyl propyle ou benzyle, et en ce que dans la formule (II'b), R4 représente un atome d'hydrogène.

Selon un autre aspect particulier de la présente invention, dans la définition du radical monovalent de formule (II'a), ou bien R1-C(=O)- représente le radical octanoyle, R3 représente l'atome d'hydrogène et R2 représente le radical méthyle, ou bien R1-C(=O)-représente le radical octanoyle, R3 représente l'atome d'hydrogène et R2 représente le radical isobutyle, ou bien R1-C(=O)- représente le radical hexadécanoyle, R3 représente l'atome d'hydrogène et R2 représente le radical isobutyle, ou bien R1-C(=O)- représente le radical octanoyle, R3 représente l'atome d'hydrogène et R2 représente le radical 1-méthyl propyle, ou bien R1-C(=O)- représente le radical hexadécanoyle, R3 représente l'atome d'hydrogène et R2 représente le radical benzyle.

Selon un autre aspect particulier de la présente invention, dans la définition du radical monovalent de formule (II'b), R1-C(=O)- représente le radical cocoyle et R4 représente un atome d'hydrogène.

Selon un aspect plus particulier l'invention a pour objet l'utilisation telle que définie précédemment, pour laquelle, dans les formules (I), (I'a) ou (I'b), s est égal à 0. Selon cet aspect particulier, le composé de formule (I') telle que définie ci-dessus est un ester du glycérol lorsque p est égal à 0, un ester de l'érythritol lorsque p est égal à 1, un ester du xylitol lorsque p est égal à 2 et un ester du sorbitol lorsque p est égal à 3.

Selon un autre aspect plus particulier, l'invention a pour objet l'utilisation telle que définie précédemment, pour laquelle, dans les formules (I'), (I'a) ou (I'b), s et p sont égaux à 0.

Selon un autre aspect plus particulier, l'invention a pour objet l'utilisation telle que définie précédemment, d'un composé de formule (I') ou d'une composition (C'1) tels que définis ci-dessus, pour laquelle dans les formules (I'), (I'a) ou (I'b), s est égal à 1. Selon cet aspect particulier, le composé de formule (I') telle que définie ci-dessus est un ester du diglycérol lorsque p est égal à 1.

Selon un autre aspect particulier, l'invention a pour objet l'utilisation telle que définie précédemment, du composé de formule (I'a) :

R'-O-CH₂-CH(OH)-[CH₂-O-CH₂]ₛ-[CH(OH)]ₚ-CH₂-O-H (I'a)

et plus particulièrement du N-octanoyl alaninate de (2,3-dihydroxy) propyle, du N-octanoyl leucinate de (2,3-dihydroxy) propyle, du N-hexadécanoyl leucinate de (2,3-dihydroxy) propyle, du N-octanoyl isoleucinate de (2,3-dihydroxy) propyle, et du N-hexadécanoyl phénylalaninate.de (2,3-dihydroxy) propyle,

Selon un autre aspect particulier, l'invention a pour objet l'utilisation telle que défini précédemment de la composition (C'1) dans laquelle le composé (la) est du N-octanoyl alaninate de (2,3-dihydroxy) propyle, du N-octanoyl leucinate de (2,3-dihydroxy) propyle, du N-hexadécanoyl leucinate de (2,3-dihydroxy) propyle, de glycérol, du N-octanoyl isoleucinate de (2,3-dihydroxy) propyle, ou du N-hexadécanoyl phénylalaninate de (2,3-dihydroxy) propyle et le composé de formule (I'b)

R-O-CH₂-CH(OH)-[CH₂-O-CH₂]ₛ-[CH(OH)]ₚ-CH₂-O-R (I'b)

est du bis(N-octanoyl alaninate) de 2-hydroxy 1,3-propanediyle, du bis(N-octanoyl leucinate) de 2-hydroxy 1,3-propanediyle, du bis(N-hexadécanoyl leucinate) de 2-hydroxy 1,3-propanediyle, du bis(N-octanoyl isoleucinate) de 2-hydroxy 1,3-propanediyle ou du bis(N-hexadécanoyl phénylalaninate) de 2-hydroxy 1,3-propanediyle.

L'invention a aussi pour objet, un procédé non thérapeutique dans le but d'empêcher ou de ralentir l'apparition des signes du vieillissement de la peau humaine ou des lèvres ou bien d'éliminer lesdits signes, comprenant au moins une étape d'application sur la peau humaine ou les lèvres, d'une formulation cosmétique à usage topique comprenant au moins un excipient cosmétiquement acceptable et une quantité efficace du composé de formule (I') ou la composition (C'1) tels que définis précédemment.

L'invention a aussi pour objet un composé de formule (I"), correspondant à la formule (I') telle que définie précédemment mais étant entendu que :
- Lorsqu'aucun des radicaux R' et R" ne représente un atome d'hydrogène, R' et R" sont identiques ils ne représentent pas non plus un radical monovalent de formule (II'a) dans laquelle le groupe R1-C(=O) représente le radical tétradécanoyle, R2 représente un radical benzyle et R3 représente un atome d'hydrogène et que ;
- Lorsque l'un des radicaux R' et R" représente un atome d'hydrogène, l'autre ne représente pas non plus un radical monovalent de formule (II'a) dans laquelle le groupe R1-C(=O) représente le radical tétradécanoyle, R2 représente un radical benzyle et R3 représente un atome d'hydrogène.

Selon un aspect particulier de la présente invention, le composé de formule (I") telle que définie ci-dessus est plus particulièrement choisi parmi les esters dérivés des acides aminés suivants : la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, l'hydroxyproline, l'hydroxylysine, la sarcosine ou l'ornithine.

Selon un aspect particulier de la présente invention, celle-ci a pour objet un composé de formule (I") telle que définie ci-dessus, pour laquelle :
- Dans la définition du radical monovalent de formule (II'a), le groupe R1-(C=O) et R3 sont tels que définis précédemment et R2 représente un radical choisi parmi les radicaux méthyle, isopropyle, isobutyle, 1-méthyl propyle ou benzyle et,
- Dans la définition du radical monovalent de formule (II'b), le groupe R1-(C=O) est tel que défini précédemment et R4 représente un atome d'hydrogène.

Selon un autre aspect particulier de la présente invention, celle-ci a pour objet un composé de formule (I') telle que définie ci-dessus dans laquelle s est égal à 0. Selon cet aspect particulier, le composé de formule (I) telle que définie ci-dessus est un ester du glycérol lorsque p représente un nombre entier égal à 0, un ester de l'érythritol lorsque p représente un nombre entier égal à 1, un ester du xylitol lorsque p représente un nombre entier égal à 2 ou un les ester du sorbitol lorsque p représente un nombre entier égal à 3. Selon cet aspect particulier, s et p sont plus particulièrement égaux à 0.

Selon un autre aspect particulier de la présente invention, celle-ci a pour objet un composé de formule (I') telle que définie ci-dessus dans laquelle s est égal à 1. Selon cet aspect particulier, s et p sont plus particulièrement égaux à 1 et le composé de formule (I') est un ester du diglycérol.

Selon un autre aspect particulier de la présente invention, celle-ci a pour objet un composé de formule (I'a) : R'-O-CH₂-CH(OH)-[CH₂-O-CH₂]ₛ-[CH(OH)]ₚ-CH₂-O-H, correspondant à la formule (I') telle que définie précédemment, dans laquelle R" représente l'atome d'hydrogène et plus particulièrement, le N-octanoyl alaninate de (2,3-dihydroxy) propyle, le N-octanoyl leucinate de (2,3-dihydroxy) propyle, le N-hexadécanoyl leucinate de (2,3-dihydroxy) propyle, le N-octanoyl isoleucinate de (2,3-dihydroxy) propyle, le N-hexadécanoyl phénylalaninate de (2,3-dihydroxy) propyle, le N-cocoyl prolinate de (2,3-dihydroxy) propyle.

Selon un autre aspect particulier de la présente invention, celle-ci a pour objet un composé de formule (I'b) : R-O-CH₂-CH(OH)-[CH₂-O-CH₂]ₛ-[CH(OH)]ₚ-CH₂-O-R, correspondant à la formule (I') telle que définie précédemment, dans laquelle R' et R" sont identiques et représentés par le radical R et plus particulièrement le bis(N-octanoyl alaninate) de 2-hydroxy 1,3-propanediyle, le bis(N-octanoyl leucinate) de 2-hydroxy 1,3-propanediyle, le bis(N-hexadécanoyl leucinate) de 2-hydroxy 1,3-propanediyle, le bis(N-octanoyl isoleucinate) de 2-hydroxy 1,3-propanediyle, le bis(N-hexadécanoyl phénylalaninate) de 2-hydroxy 1,3-propanediyle, le bis(N-cocoyl prolinate) de 2-hydroxy 1,3-propanediyle.

L'invention a aussi pour objet une composition (C"1) comprenant pour 100% de sa masse :
- De 99% massique à 20% massique d'au moins un composé de formule (I"a) :

   R'-O-CH₂-CH(OH)-[CH₂-O-CH₂]ₛ-[CH(OH)]ₚ-CH₂-O-H (I"a),

   formule (I"a) correspondant à la formule (I") telle que définie à la revendication 7, dans laquelle R" représente un atome d'hydrogène,
- De 1% massique à 80% massique d'au moins un composé de formule (I"b) :

   R-O-CH₂-CH(OH)-[CH₂-O-CH₂]ₛ-[CH(OH)]ₚ-CH₂-O-R (I"b),

   formule (I"b) correspondant à la formule (I") telle que définie à la revendication 7, dans laquelle R' et R" sont identiques et représentés par le radical R ; plus particulièrement une composition (C"1) telle que définie ci-dessus, Dans la définition du radical monovalent de formule (II'a), le groupe R1-(C=O) et R3 sont tels que définis précédemment et R2 représente un radical choisi parmi les radicaux méthyle, isopropyle, isobutyle, 1-méthyl propyle ou benzyle et, dans la définition du radical monovalent de formule (II'b), le groupe R1-(C=O) est tel que défini précédemment et R4 représente un atome d'hydrogène et/ou une composition (C"1) telle que définie ci dessus pour laquelle, dans les formules (I"a) et (I"b), ou bien s et p sont égaux à 0, ou bien s et p sont égaux à 1.

Le composé de formule (I') telle que définie précédemment peut être préparé selon un procédé de préparation comprenant :
- Une étape a) d'estérification, soit d'un composé de formule (IV'a) : 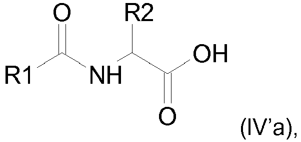 dans laquelle R₁-C(=O)- et R2 sont tels que définis dans la formule (II'a), soit d'un composé de formule (IV'b) : 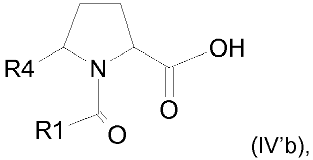 dans laquelle R₁-C(=O)- et R4 sont tels que définis pour la formule (II'b), avec un composé de formule (V) ; H-O-CH₂-CH(OH)-[CH₂-O-CH₂]ₛ-[CH(OH)]ₚ-CH₂-O-H, dans laquelle dans laquelle s est un nombre entier égal à 0 ou à 1, et p est un nombre entier égal à 0, 1, 2 ou 3, pour obtenir, soit le composé de formule (l'a), soit le composé de formule (I'b), soit un mélange (M) du composé de formule (l'a) et du composé de formule (I'b) ; et si nécessaire ou si désiré,
- Une étape b) de séparation des composés de formule (l'a) et de formule (I'b), à partir du dudit mélange (M) obtenu à l'étape (a).

Les composés de formules (IV'a) et (IV'b) sont connus ou sont synthétisables par N-acylation des α-amino acides correspondant selon des méthodes connues de l'homme du métier.

Dans le procédé tel que défini ci-dessus, le rapport molaire composé de formule (IV'a) ou de formule (IV'b) sur composé de formule (V') est généralement compris entre 3/1 et 1/5, plus particulièrement entre 1/1 et 1/5, et encore plus particulièrement entre 1/1 et 1/3.

Dans le procédé tel que défini ci-dessus, l'étape b) de séparation des composés de formule (l'a) et de formule (I'b) est mise en oeuvre par les méthodes classiques de séparation connues de l'homme du métier.

Le composé de formule (I') telle que définie précédemment peut être également préparé selon un procédé de préparation comprenant :.
- Une étape a1) d'estérification, soit d'un composé de formule (IV'a) 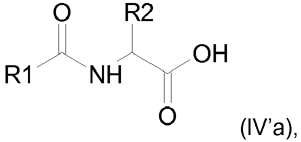 dans laquelle R₁-C(=O)- et R2 sont tels que définis dans la formule (II'a), soit d'un composé de formule (IV'b) : 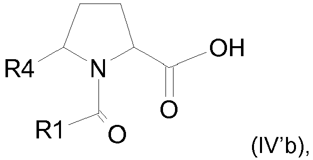 dans laquelle R₁-C(=O)- et R4 sont tels que définis pour la formule (II'b), avec un alcool de formule (VI') :

   R5-OH (VI'),

   dans laquelle R5 représente un radical aliphatique linéaire comportant de 1 à 4 atomes de carbone, pour former :
- Soit un composé de formule (VII'a) : 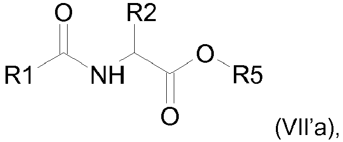 dans laquelle R₁-C(=O)-, R2 et R5 sont tels que définis précédemment, soit un composé de formule (VII'b) : 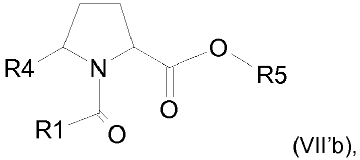 dans laquelle R₁-C(=O)-, R4 et R5 sont tels que définis précédemment ;
   - Une étape a2) de trans-estérification du composé de formule (VII'a) ou du composé de formule (VII'b) obtenu à l'étape a1), par réaction avec le composé de formule (V'), pour obtenir, soit le composé de formule (la), soit le composé de formule (I'b), soit un mélange (M) du composé de formule (l'a) et du composé de formule (I'b) ; et si nécessaire ou si désiré,
   - La mise en oeuvre de l'étape b).

Dans le procédé tel que décrit ci-dessus, l'étape a1) est généralement réalisée à une température d'environ comprise entre 60°C et 120°C, sous gaz inerte, et en présence d'un système catalytique acide. Par système catalytique acide on désigne les acides forts comme l'acide sulfurique, l'acide chlorhydrique, l'acide phosphorique, l'acide nitrique, l'acide hypophosphoreux, l'acide méthanesulfonique, l'acide para-toluène sulfonique, l'acide trifluorométhane sulfonique, ou les résines échangeuses d'ions acides.

Dans l'étape a1) de la variante du procédé tel que décrit ci-dessus, le rapport molaire composé de formule (IV'a) ou composé de formule (IV'b) sur alcool de formule (VI') est généralement compris entre 1/1 et 1/10, plus particulièrement entre 1/1 et 1/8, et encore plus particulièrement entre 1/2 et 1/8.

Dans la variante du procédé de préparation des composés de formule (I') telle que décrite ci-dessus, l'étape a2) de trans-estérification de l'ester de formule (VII'a) ou de formule (VII'b) obtenu à l'étape a1) est généralement réalisée à une température d'environ comprise entre 80°C et 180°C, plus particulièrement comprise entre 100°C et 150°C, encore plus particulièrement entre 120°C et 150°C, sous gaz inerte, et en présence d'un système catalytique acide tel que précédemment décrit, et avec distillation sous vide de l'alcool de formule (VI) formé in-situ.

Dans l'étape a2) de cette variante du procédé de préparation des composés de formule (I) telle que décrite ci-dessus, le rapport molaire composé de formule (VIIa) et/ou de formule (VII'b) sur le composé de formule (V') est compris entre 3/1 et 1/5, plus particulièrement entre 1/1 et 1/5, et encore plus particulièrement entre 1/1 et 1/3.

La composition (C'1) dont l'utilisation telle que précédemment définie est l'objet de l'invention et la.composition (C"1) telle que précédemment définie, peuvent être préparéee par diverses voies.

Une première voie de préparation des compositions (C'1) et (C"1) consiste à mélanger dans les proportions massiques souhaitées, le composé de formule (I'a) ou le mélange de composés de formule (I'a) telle que définie ci-dessus, avec le composé de formule (Ib), ou le mélange de composés de formule (I'b) telle que définie ci-dessus.

Une seconde voie de préparation de ces compositions est le procédé de préparation du composé de formule (I') tel que décrit ci-dessus, en faisant réagir dans les proportions souhaitées, le composé de formule (V') avec le composé de formule (IV'a) ou de formule (IV'b) ou un mélange de ces deux composés.

Une troisième voie de préparation des compositions (C'1) et (C"1) est la variante du procédé de préparation du composé de formule (I') telle que décrite ci-dessus, en faisant réagir dans les proportions souhaitées, le composé de formule (V') avec le composé de formule (VII'a) ou de formule (VII'b) ou un mélange de ces deux composés.

L'invention a aussi pour objet une formulation cosmétique à usage topique caractérisée en ce qu'elle comprend au moins un excipient cosmétiquement acceptable et une quantité efficace du composé de formule (I") ou de la composition (C"1) tels que définis ci-dessus.

Le composé de formule (I"), et la composition (C"1), sont mis en oeuvre au en une quantité efficace au sein d'une formulation cosmétique et administrés par voie orale, topique ou parentérale, et plus particulièrement par voie topique.

L'invention a aussi pour objet un procédé non thérapeutique dans le but d'empêcher ou de ralentir l'apparition des signes du vieillissement de la peau humaine ou des lèvres ou bien d'éliminer lesdits signes, comprenant au moins une étape d'application sur la peau humaine ou les lèvres, d'une formulation cosmétique à usage topique comprenant au moins un excipient cosmétiquement acceptable et une quantité efficace d'au moins un composé de formule (I") ou de la composition (C"1) tels que définis précédemment.

Les expressions « à usage topique » ou « par voie topique » utilisées ci-dessus signifient que ladite formulation cosmétique, est mise en oeuvre par application sur la peau, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact. avec la peau.

L'expression « cosmétiquement acceptable » utilisée dans la définition de la formulation cosmétique telle que décrite ci-dessus, signifie selon la directive du Conseil de la Communauté Economique Européenne N°76/768/CEE du 27 juillet 1976 modifiée par la directive N°93/35/CEE du 14 juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par quantité efficace de composé de formule (I) ou de composition (C'1) ou (C"1) tels que définis précédemment, on entend, pour 100% de la masse de ladite formulation cosmétique à usage topique, la quantité comprise entre 0,1% et 5% massique, plus particulièrement entre 0,1% et 3% massique, et encore plus particulièrement entre 0,5% et 2% massique de composé de formule (I) ou de composition (C'1) ou (C"1).

Les formulations cosmétiques à usage topique mises en oeuvre dans le procédé cosmétique tel que défini ci-dessus, se présentent généralement sous forme de solutions aqueuses ou hydro-alcooliques ou hydro-glycoliques, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre. Elles peuvent être conditionnées dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, le composé de formule (I') ou (I") ou à la composition (C'1) ou (C"1), présents dans les formulations cosmétiques à usage topique mises en oeuvre dans le procédé cosmétique objet de la présente invention, est associé à des additifs chimiques habituellement mis en oeuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer au composé de formule (I') ou (I") à la composition (C'1) ou (C"1) dans les formulations cosmétiques à usage topique mises en oeuvre dans le procédé cosmétique objet de la présente invention, on peut citer les tensioactifs moussants et/ou détergents anioniques, cationiques, amphotères ou non ioniques.

Parmi les tensioactifs anioniques moussants et/ou détergents que l'on peut associer au composé de formule (I') ou (I") à la composition (C'1) ou (C"1) dans les formulations cosmétiques à usage topique mises en oeuvre dans le procédé cosmétique objet de la présente invention, on peut citer les sels de métaux alcalins, de métaux alcalino-terreux, d'ammonium, d'amines, ou d'aminoalcools, d'alkylethersulfates, d'alkylsulfates, d'alkylamidoéthersulfates, d'alkylarylpolyéthersulfates, de monoglycérides sulfates, d'alpha-oléfinesulfonates, de paraffinessulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines ou de dérivés N-acylés d'acides gras.

Parmi les tensioactifs amphotères moussants et/ou détergents que l'on peut associer au composé de formule (I') ou (I") à la composition (C'1) ou (C"1) dans les formulations cosmétiques à usage topique mises en oeuvre dans le procédé cosmétique objet de la présente invention, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut associer au composé de formule (I') ou (I") à la composition (C'1) ou (C"1) dans les formulations cosmétiques à usage topique mises en oeuvre dans le procédé cosmétique objet de la présente invention, on peut citer particulièrement les dérivés d'ammoniums quaternaires.

Parmi les tensioactifs non ioniques moussants et/ou détergents que l'on peut associer au composé de formule (I') ou (I") à la composition (C'1) ou (C"1) dans les formulations cosmétiques à usage topique mises en oeuvre dans le procédé cosmétique, objet de la présente invention, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1,12-dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les esters gras d'alkyl polyglycosides éventuellement poly(alcoxylés), comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide-2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaires, branchés ou réticulés d'au moins un monomère possédant une fonction acide fort, libre, partiellement salifiée ou totalement salifiée, avec au moins un monomère neutre, et au moins un monomère de formule (VIII) :

CH₂=C(R6)-C(=O)-[CH₂-CH₂-O]ₙ-R7 (VIII)

dans laquelle R6 représente un atome d'hydrogène ou un radical méthyle, R7 représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer au composé de formule (I') ou (I") à la composition (C'1) ou (C"1) dans les formulations cosmétiques à usage topique mises en oeuvre dans le procédé cosmétique objet de la présente invention, peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés que l'on peut associer au composé de formule (I') ou (I") à la composition (C'1) ou (C"1) dans les formulations cosmétiques à usage topique mises en oeuvre dans le procédé cosmétique objet de la présente invention, peuvent être sélectionnés parmi les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants que l'on peut associer au composé de formule (I') ou (I") à la composition (C'1) ou (C"1) dans les formulations cosmétiques à usage topique mises en oeuvre dans le procédé cosmétique, objet de la présente invention, on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer au composé de formule (I') ou (I") à la composition (C'1) ou (C"1) dans les formulations cosmétiques à usage topique mises en oeuvre dans le procédé cosmétique objet de la présente invention, on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer au composé de formule (I') ou (I") à la composition (C'1) ou (C"1) dans les formulations cosmétiques à usage topique mises en oeuvre dans le procédé cosmétique objet de la présente invention, on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer au composé de formule (I') ou (I") à la composition (C'1) ou (C"1) dans les formulations cosmétiques à usage topique mises en oeuvre dans le procédé cosmétique objet de la présente invention, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer au composé de formule (I') ou (I") à la composition (C'1) ou (C"1) dans les formulations cosmétiques à usage topique mises en oeuvre dans le procédé cosmétique objet de la présente invention, on peut citer les xylènes sulfonates, les cumène sulfonates, l'hexyl polyglucoside, le (2-éthyl hexyl) polyglucoside, le n-heptyl polyglucoside.

Comme exemples d'agents tensioactifs émulsionnants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les tensioactifs non ioniques, des tensioactifs anioniques, des tensioactifs cationiques.

Comme exemples de tensioactifs non-ioniques émulsionnants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécylpolyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkyl polyglycosides tels que décrits précédemment.

Comme exemples de tensioactifs anioniques que l'on peut associer au composé de formule (I') ou (I") à la composition (C'1) ou (C"1) dans les formulations cosmétiques à usage topique mises en oeuvre dans le procédé cosmétique, objet de la présente invention, on peut citer le glycéryl stéarate citrate, le cétéarylsulfate, les savons comme le stéarate de sodium ou le stéarate de triéthanolammonium, les dérivés N-acylés d'acides aminés salifiés par exemple le stéaroyl glutamate.

Comme exemples de tensioactifs cationiques émulsionnants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer au composé de formule (I') ou (I") à la composition (C'1) ou (C"1) dans les formulations cosmétiques à usage topique mises en oeuvre dans le procédé cosmétique objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer au composé de formule (I') ou (I") à la composition (C'1) ou (C"1) dans les formulations cosmétiques à usage topique mises en oeuvre dans le procédé cosmétique objet de la présente invention, on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples d'agents déodorants que l'on peut associer au composé de formule (I') ou (I") à la composition (C'1) ou (C"1) dans les formulations cosmétiques à usage topique mises en oeuvre dans le procédé cosmétique objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.
Comme exemples d'huiles que l'on peut associer au composé de formule (I') ou (I") à la composition (C'1) ou (C"1) dans les formulations cosmétiques à usage topique mises en oeuvre dans le procédé cosmétique objet de la présente invention, on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer au composé de formule (I') ou (I") à la composition (C'1) ou (C"1) dans les formulations cosmétiques à usage topique mises en oeuvre dans le procédé cosmétique objet de la présente invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer au composé de formule (I') ou (I") à la composition (C'1) ou (C"1) dans les formulations cosmétiques à usage topique mises en oeuvre dans le procédé cosmétique objet de la présente invention, on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE™MSH, le SEPICALM™VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, comme la dihydroxyacétone, l'isatine, l'alloxane, la ninhydrine, le glycéraldéhyde, l'aldéhyde mésotartrique, la glutaraldéhyde, l'érythrulose.

Comme exemples d'agents antioxydants que l'on peut associer au composé de formule (I') ou (I") à la composition (C'1) ou (C"1) dans les formulations cosmétiques à usage topique mises en oeuvre dans le procédé cosmétique objet de la présente invention, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le nom INCI : Tetrasodium Glutamate Diacetate.

Comme exemples de filtres solaires que l'on peut associer au composé de formule (I') ou (I") à la composition (C'1) ou (C"1) dans les formulations cosmétiques à usage topique mises en oeuvre dans le procédé cosmétique objet de la présente invention, on peut citer tous ceux figurant dans la directive cosmétique 76/768/CEE modifiée annexe VII.

Parmi les filtres organiques solaires que l'on peut associer au composé de formule (I') ou (I") à la composition (C'1) ou (C"1) dans les formulations cosmétiques à usage topique mises en oeuvre dans le procédé cosmétique objet de la présente invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxy éthyle, le cinnamate de p-méthoxy cyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", que l'on peut associer au composé de formule (I') ou (I") à la composition (C'1) ou (C"1) dans les formulations cosmétiques à usage topique mises en oeuvre dans le procédé cosmétique objet de la présente invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a aussi pour objet un composé de formule (I') ou (I"), ou une composition (C'1) ou (C"1), pour son utilisation dans une méthode de traitement thérapeutique des signes du vieillissement de la peau humaine ou des lèvres appliquée au corps humain.

Les exemples suivants illustrent l'invention sans toutefois la limiter.

### Exemple 1 : préparation d'une composition (C1_{A}) selon l'invention

On introduit 500 grammes d'alanine, soit un équivalent molaire, dans un mélange hydro-alcoolique constitué de 1.800 grammes d'eau et de 200 grammes d'isopropanol à une température de 20°C. Le pH du milieu est ajusté à 10 par ajout d'une solution de soude à 30%. 731,7 grammes de chlorure d'octanoyle, soit 0,8 équivalent molaire, sont ensuite versés progressivement dans le mélange maintenu entre 20°C et 50°C et à un pH entre 10 et 10,5.

Le milieu réactionnel est ensuite maintenu sous agitation pendant deux heures puis chauffé jusqu'à atteindre 70°C, puis additionné de 979,6 grammes d'une solution acide d'acide phosphorique à 75% pour atteindre progressivement une valeur de pH égale à 2,0. La phase aqueuse du milieu est décantée et soutirée et la phase organique restant dans le réacteur est lavée plusieurs fois à la saumure à température ambiante sous agitation. A l'issue du lavage, puis du séchage par distillation sous vide de l'eau résiduelle, on obtient la phase organique comprenant 922,3 grammes de N-octanoyl alanine souhaité.

59,2 grammes de glycérol soit un équivalent molaire de glycérol, sont introduits sous agitation dans le réacteur comprenant une fraction de la phase organique précédente, dans laquelle se trouve 0,8 équivalent molaire de N-octanoyl alanine. La température est portée à 120°C. 0,44 gramme d'acide sulfurique à 98% et 0,44 gramme d'acide hypophosphoreux à 50% sont ensuite ajoutés et le mélange résultant porté à 125°C, sous vide partiel avec barbotage d'azote. Le mélange réactionnel est ensuite maintenu pendant 12 heures sous agitation à cette température, puis neutralisé par ajout d'une solution de soude à 30% de façon à obtenir un pH de la composition (C1_{A}), diluée à 5% dans l'eau, compris entre 3,0 et 6,0.

Les caractéristiques analytiques de la composition (C1_{A}) obtenue sont les suivantes :
Indice acide (selon NFT 60-204) = 16,8
pH 5% de la composition (C1_{A}) dans l'eau (selon la méthode NFT 73-206) = 4,5
Indice d'Hydroxyle (selon U.S. Pharmacopeia XXI NF XVI 01/011985) = 284,0
Indice d'ester (calculé par la différence entre l'indice de saponification mesuré selon la méthode NFT 60-110 et l'indice d'acide mesuré selon la méthode NFT 60-204) = 196,2 Indice de saponification (selon NFT 60-206) = 213,0 mg KOH/g

### Exemple 2 : préparation d'une composition (C1_{B}) selon l'invention.

Le mode opératoire du procédé décrit dans l'exemple 1 est mis en oeuvre en remplaçant l'équivalent molaire d'alanine par un équivalent molaire de leucine, pour obtenir la composition (C1_{B}) dont les caractéristiques analytiques sont les suivantes :
Indice acide (selon NFT 60-204) = 13,7
pH 5% de la composition (C1_{B}) dans l'eau (selon la méthode NFT 73-206) = 5,5
Indice d'Hydroxyle (selon U.S. Pharmacopeia XXI NF XVI 01/011985) = 316,9
Indice d'ester (calculé par la différence entre l'indice de saponification mesuré selon la méthode NFT 60-110 et l'indice d'acide mesuré selon la méthode NFT 60-204) = 164,9 Indice de saponification (selon NFT 60-206) = 178,6 mg KOH/g

### Exemple 3 : préparation d'une composition (C1_{C}) selon l'invention.

Le mode opératoire du procédé décrit dans l'exemple 1 est mis en oeuvre en remplaçant l'équivalent molaire d'alanine par un équivalent molaire de leucine et le 0,8 équivalent molaire de chlorure d'octanoyle par 0,8 équivalent molaire de chlorure d'hexadécanoyle pour obtenir la composition (C1_{c}) dont les caractéristiques analytiques sont les suivantes :
Indice acide (selon NFT 60-204) = 12,2
pH 5% de la composition (C1_{C}) dans l'eau (selon la méthode NFT 73-206) = 6,4
Indice d'Hydroxyle (selon U.S. Pharmacopeia XXI NF XVI 01/011985) = 150,7
Indice d'ester (calculé par la différence entre l'indice de saponification mesuré selon la méthode NFT 60-110 et l'indice d'acide mesuré selon la méthode NFT 60-204) = 132,5 Indice de saponification (selon NFT 60-206) = 144,7 mg KOH/g

### Exemple 4 : préparation d'une composition (C1_{D}) selon l'invention.

Le mode opératoire du procédé décrit dans l'exemple 1 est mis en oeuvre en remplaçant l'équivalent molaire d'alanine par un équivalent molaire d'isoleucine, pour obtenir la composition (C1_{D}) dont les caractéristiques analytiques sont les suivantes :
Indice acide (selon NFT 60-204) = 44,8
pH 5% de la composition (C1_{D}) dans l'eau (selon la méthode NFT 73-206) = 5,1
Indice d'Hydroxyle (selon U.S. Pharmacopeia XXI NF XVI 01/011985) = 364,7
Indice d'ester (calculé par la différence entre l'indice de saponification mesuré selon la méthode NFT 60-110 et l'indice d'acide mesuré selon la méthode NFT 60-204) = 142,9 Indice de saponification (selon NFT 60-206) = 187,7 mg KOH/g

### Exemple 5 : préparation d'une Composition (C1_{E}) selon l'invention

Le mode opératoire du procédé décrit dans l'exemple 1 est mis en oeuvre en remplaçant l'équivalent molaire d'alanine par un équivalent molaire de phénylalanine et le 0,8 équivalent molaire de chlorure d'octanoyle par 0,8 équivalent molaire de chlorure d'hexadécanoyle pour obtenir la composition (C1_{E}) dont les caractéristiques analytiques sont les suivantes :
Indice acide (selon NFT 60-204) = 12,4
pH 5% de la composition (C1_{E}) dans l'eau (selon la méthode NFT 73-206) = 6,0
Indice d'Hydroxyle (selon U.S. Pharmacopeia XXI NF XVI 01/011985) = 230,1
Indice d'ester (calculé par la différence entre l'indice de saponification mesuré selon la méthode NFT 60-110 et l'indice d'acide mesuré selon la méthode NFT 60-204) = 112,9 Indice de saponification (selon NFT 60-206) = 125,3 mg KOH/g

### Exemple 6 : préparation d'une Composition (C1_{F}) selon l'invention.

Le mode opératoire du procédé décrit dans l'exemple 1 est mis en oeuvre en remplaçant l'équivalent molaire d'alanine par un équivalent molaire de proline et le 0,8 équivalent molaire de chlorure d'octanoyle par 0,8 équivalent molaire de chlorure de cocoyle pour obtenir la composition (C1_{F}) dont les caractéristiques analytiques sont les suivantes :
Indice acide (selon NFT 60-204) = 28,7
pH 5% de la composition (C1_{E}) dans l'eau (selon la méthode NFT 73-206) = 5,1
Indice d'Hydroxyle (selon U.S. Pharmacopeia XXI NF XVI 01/011985) = 283,6
Indice d'ester (calculé par la différence entre l'indice de saponification mesuré selon la méthode NFT 60-110 et l'indice d'acide mesuré selon la méthode NFT 60-204) = 138,0 Indice de saponification (selon NFT 60-206) = 166,7 mg KOH/g

### Mise en évidence de l'activité antivieillissement des composés et des compositions selon l'invention par une étude in vitro de la migration de fibroblastes humains.

La mise en évidence de l'activité antivieillissement des compositions selon l'invention a été réalisée par la mise en oeuvre d'un modèle d'étude consistant à étudier la capacité migratoire des fibroblastes humains normaux traités ou non avec les compositions selon l'invention et des références. Le test de migration des fibroblastes est un test couramment utilisé dans le secteur de la cosmétique et de la pharmacie et décrit notamment dans la demande internationale publiée sous le numéro WO 2010/056908 A1. Il permet de reproduire *in vitro* le phénomène de migration des fibroblastes ; une diminution des capacités migratoires des fibroblastes étant rapportée comme associée au vieillissement cutané (1) (4).

### Protocole :

Des Fibroblastes Humains normaux au passage R5 ont été amplifiés en flasque de culture T75, puis ensemencés à 25 000 cellules/puits en plaques de culture spécifiques. Celles-ci sont dédiées à la migration (Oris™ de Platypus), intégrant une zone centrale sans dépôt initial de cellules (stoppers).Après 96 heures d'amplification en plaque, et 2 heures avant le traitement, tous les puits ont été traités avec de la Mitomycine C (2h à 10µg/ml), afin de stopper la prolifération cellulaire, et ainsi n'observer que le phénomène de migration. Les stoppeurs ont été retirés, et les références, les produits, ou du milieu standard pour Fibroblastes à 2% de Serum de Veau Foetal (SVF) ont été appliquées sur les cellules sous 100µl, puis incubées pendant 40h à 37°C sous 5% de CO2. Chaque condition a été réalisée en quadriplicate

### Evaluation des effets :

A l'issue de l'incubation, un marquage des cellules à la Calcein AM a été réalisé (5µM, 20 minutes à 37°C), afin de visualiser les cellules viables en fluorescence (révélation du cytoplasme). Des photos on été réalisées, après avoir inséré un cache noir sous les plaques de culture, afin de ne visualiser que la zone où les cellules n'ont pas été déposées. Ainsi, seules les cellules ayant migrées ont été photographiées (objectif x4 ; avec un réducteur sur l'adaptateur de la caméra x 0,7). La distance de migration moyenne des cellules (D_{migr}).a été mesurée avec NIS-Elements-BR 3.0 (4 cellules). Les moyennes et écart-types des distances ont été calculées sur les quadriplicates. Les pourcentages d'effet migratoire par rapport au témoin, ainsi que les statistiques (test de Student) ont également été calculés.

### Résultats :

Les résultats obtenus sont consignés dans le Tableau 1 ci-dessous :

**Tableau 1: Migration de fibroblastes en présence des compositions testés**

| **Produits testés (p/v)** | **(D_{migr}) en µm** | **[(D_{migr}) - (D_{migr})_{témoin}]/(D_{migr})_{témoin}** |
|---|---|---|
| Témoin de culture SVF (20mg/ml) | 168 ± 34 | |
| Epidermal Growth Factor (EGF) 10ng/ml | 203 ± 20 | + 21% |
| Epidermal Growth Factor (EGF) 50ng/ml | 212 ± 8 | + 26% |
| Composition (C1_{A}) (5µg/ml) | 203 ± 34 | + 21% |
| Composition (C1_{A}) (10µg/ml) | 198 ± 23 | + 18% |
| Composition (C1_{B}) (5µg/ml) | 185 ± 17 | + 10% |
| Composition (C1_{C}) (5µg/ml) | 190 ± 6 | + 13% |
| Composition (C1_{C}) (10µg/ml) | 185 ± 3 | + 10% |
| Composition (C1_{D}) (10µg/ml) | 181 ± 7 | + 8% |
| Composition (C1_{E}) (5µg/ml) | 192 ± 9 | + 14% |
| Composition (C1_{F}) (5µg/ml) | 195 ± 6 | + 16% |

La combinaison d'EGF, à 10 et 50ng/ml, avec les fibroblastes humains normaux montrent une augmentation de la distance de migration desdits fibroblastes, validant ainsi le modèle choisi.

La combinaison des compositions (C1_{A}), (C1_{B}), (C1_{C}), (C1_{D}), (C1_{E}) et (C1_{F}) avec les fibroblastes humains normaux montrent une augmentation significative de la distance de migration desdits fibroblastes, et par conséquent une amélioration des propriétés migratoires des fibroblastes du derme de la peau humain, constituant ainsi un moyen efficace de prévenir et/ou traiter le vieillissement de la peau du corps humain et des lèvres.

### Bibliographie

(1) : Schulze et al., "Stiffening of human skin fibroblasts with age; Clin. Plast. Surg.; 2012 ; 39(1):9-20.
(2) : Baraibar et Friguet, "Oxidative proteome modifications target specific cellular pathways during oxidative stress, cellular senescence and ageing" ; Exp Gerontol; 2013; 48(7):620-5.
(3) : Kondo et al., "Inhibitory effects of human serum on human fetal skin fibroblast migration: migration-inhibitory activity and substances in serum, and its age-related changes"; In Vitro Cell Dev Biol Anim; 2000 ; 36(4):256-61.
(4) : Jang et al., Prolonged activation of ERK contributes to the photorejuvenation effect in photodynamis therapy in human dermal fibroblasts ; JID ; 2013 ; 133(9):2265-75.
(5) : Houreld et Abrahamse « Low-intensity laser irradiation stimulates wound healing in diabetic wounded fibroblast cells (WS1) »,.2010, Diabetes Technol Ther, Dec;12(12*)*
(6) : Tang et al., « A rice-derived recombinant human lactoferrin stimulates fibroblast proliferation, migration, and sustains cell survival », 2010, Wound Repair Regen, Jan-Feb 18(1*)*
(7) Demirovic et Rattan,« Curcumin induces stress response and hormetically modulates wound healing ability of human skin fibroblasts undergoing ageing in vitro », 2011, Biogerontology, Mar 6
(8) : Rojo et al., « Wound healing properties of nut oil from Pouteria lucuma », 2010, J Cosmet Dermatol, Sep 9(3*)*

## Revendications

1. Utilisation non thérapeutique d'un composé de formule (I') :
R'-O-CH₂-CH(OH)-[CH₂-O-CH₂]ₛ-[CH(OH)]ₚ-CH₂-O-R" (I'),
formule (I') dans laquelle s est égal à 0 ou à 1, et p est égal à 0, 1, 2 ou 3, R' et R", identiques ou différents, représentent soit un atome d'hydrogène, soit un radical monovalent de formule (II'a) : 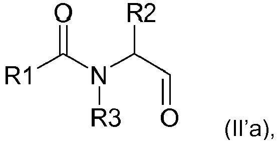 formule (II'a) dans laquelle le groupe R1-C(=O)- représente un radical choisi parmi les radicaux octanoyle, décanoyle, ω-undécylènoyle, dodécanoyle, tétradécanoyle, hexadécanoyle, octadécanoyle, cocoyle, eicosanoyle, 9-octadécènoyle, éicosènoyle, 9,12-octadécadiènoyle ou 9,12,15-octadécatriénoyle, R2 représente un atome d'hydrogène ou un radical choisi parmi les radicaux méthyle, isopropyle, isobutyle, 1-méthyl propyle, hydroxyméthyle, 1-hydroxy éthyle, 4-aminobutyle, 3-guanidino propyle, 3-uréido propyle, (1-amino carbonyl) méthyle, carboxy méthyle, 2-carboxy éthyle, 2-(amino carbonyl) éthyle, benzyle, 4-hydroxy benzyle, 3,4-dihydroxy benzyle, [1H-indol-3-yl]méthyle, (1H-imidazol-4-yl) méthyle, 3-amino propyle, et R3 représente un atome d'hydrogène ou un radical méthyle ; soit un radical monovalent de formule (II'b) : 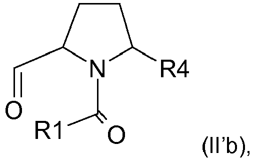 formule (II'b) dans laquelle le groupe R₁-C(=O)- représente un radical choisi parmi radicaux représente un radical choisi parmi les radicaux octanoyle, décanoyle, ω-undécylènoyle, dodécanoyle, tétradécanoyle, hexadécanoyle, octadécanoyle, cocoyle, eicosanoyle, 9-octadécènoyle, éicosènoyle, 9,12-octadécadiènoyle ou 9,12,15-octadécatriénoyle et R4 représente un atome d'hydrogène ou un radical hydroxy,
étant entendu :
- qu'au moins un des radicaux R' ou R" ne représente pas un atome d'hydrogène,
- que lorsqu'aucun des radicaux R' et R" ne représente un atome d'hydrogène, R' et R" sont identiques mais ne représentent pas un radical monovalent de formule (II'a) dans laquelle le groupe R1-C(=O) représente le radical ω-undécylènoyle, R2 représente un radical benzyle et R3 représente un atome d'hydrogène et
- que lorsque l'un des radicaux R' et R" représente un atome d'hydrogène, l'autre ne représente pas un radical monovalent de formule (II'a) dans laquelle le groupe R1-C(=O) représente le radical ω-undécylènoyle, R2 représente un radical benzyle et R3 représente un atome d'hydrogène,
ladite utilisation étant dans le but d'empêcher ou de ralentir l'apparition des signes du vieillissement de la peau humaine ou des lèvres ou bien d'éliminer lesdits signes.

2. Utilisation telle que définie à la revendication 1, d'une composition (C'1) comprenant pour 100% de sa masse :
- De 99% massique à 20% massique d'au moins un composé de formule (I'a) :
R'-O-CH₂-CH(OH)-[CH₂-O-CH₂]ₛ-[CH(OH)]ₚ-CH₂-O-H (I'a),
formule (I'a) correspondant à la formule (I') telle que définie précédemment dans laquelle R" représente un atome d'hydrogène,
- De 1% massique à 80% massique d'au moins un composé de formule (I'b) :
R-O-CH₂-CH(OH)-[CH₂-O-CH₂]ₛ-[CH(OH)]ₚ-CH₂-O-R (Ib),
formule (Ib) correspondant à la formule (I') telle que définie précédemment dans laquelle R' et R" sont identiques et représentés par le radical R.

3. Utilisation telle que définie à l'une quelconque des revendications 1 ou 2, **caractérisée en ce que** dans la formule (II'a), R2 représente un radical choisi parmi, les radicaux méthyle, isopropyle, isobutyle, 1-méthyl propyle ou benzyle, et **en ce que** dans la formule (II'b), R4 représente un atome d'hydrogène.

4. Utilisation telle que définie à l'une quelconque des revendications 1 à 3 pour laquelle, dans les formules (I'), (I'a) ou (I'b) ou bien s et p sont égaux à 0 ou bien s et p sont égaux à 1.

5. Procédé non thérapeutique dans le but d'empêcher ou de ralentir l'apparition des signes du vieillissement de la peau humaine ou des lèvres ou bien d'éliminer lesdits signes, comprenant au moins une étape d'application sur la peau humaine ou les lèvres, d'une formulation cosmétique à usage topique comprenant au moins un excipient cosmétiquement acceptable et une quantité efficace du composé de formule (I'), telle que définie à l'une quelconques des revendications 1 à 4.

6. Procédé non thérapeutique dans le but d'empêcher ou de ralentir l'apparition des signes du vieillissement de la peau humaine ou des lèvres ou bien d'éliminer lesdits signes, comprenant au moins une étape d'application sur la peau humaine ou les lèvres, d'une formulation cosmétique à usage topique comprenant au moins un excipient cosmétiquement acceptable et une quantité efficace de la composition (C'1) telle que définie à l'une quel conque des revendications 2 à 4.

7. Composé de formule (I"), correspondant à la formule (I') telle que définie à la revendication 1 mais étant entendu que :
- Lorsqu'aucun des radicaux R' et R" ne représente un atome d'hydrogène et que R' et R" sont identiques, ils ne représentent pas non plus un radical monovalent de formule (II'a) dans laquelle le groupe R1-C(=O) représente le radical tétradécanoyle, R2 représente un radical benzyle et R3 représente un atome d'hydrogène et que ;
- Lorsque l'un des radicaux R' et R" représente un atome d'hydrogène, l'autre ne représente pas non plus un radical monovalent de formule (II'a) dans laquelle le groupe R1-C(=O) représente le radical tétradécanoyle, R2 représente un radical benzyle et R3 représente un atome d'hydrogène.

8. Composé de formule (I") telle que définie à la revendication 7, pour laquelle :
- Dans la définition du radical monovalent de formule (II'a), le groupe R1-(C=O) et R3 sont tels que définis précédemment et R2 représente un radical choisi parmi les radicaux méthyle, isopropyle, isobutyle, 1-méthyl propyle ou benzyle et,
- Dans la définition du radical monovalent de formule (II'b), le groupe R1-(C=O) est tel que défini précédemment et R4 représente un atome d'hydrogène.

9. Composé de formule (I") telle que définie à l'une des revendications 7 ou 8, dans laquelle, ou bien s et p sont égaux à 0, ou bien s et p sont égaux à 1.

10. Composition (C"1) comprenant pour 100% de sa masse :
- De 99% massique à 20% massique d'au moins un composé de formule (I"a) :
R'-O-CH₂-CH(OH)-[CH₂-O-CH₂]ₛ-[CH(OH)]ₚ-CH₂-O-H (I"a),
formule (I"a) correspondant à la formule (I") telle que définie à la revendication 7, dans laquelle R" représente un atome d'hydrogène,
- De 1% massique à 80% massique d'au moins un composé de formule (I"b) :
R-O-CH₂-CH(OH)-[CH₂-O-CH₂]ₛ-[CH(OH)]ₚ-CH₂-O-R (I"b),
formule (I"b) correspondant à la formule (I") telle que définie à la revendication 7, dans laquelle R' et R" sont identiques et représentés par le radical R.

11. Composition (C"1) telle que définie à la revendication 10 pour laquelle :
- Dans la définition du radical monovalent de formule (II'a), le groupe R1-(C=O) et R3 sont tels que définis précédemment et R2 représente un radical choisi parmi les radicaux méthyle, isopropyle, isobutyle, 1-méthyl propyle ou benzyle et,
- Dans la définition du radical monovalent de formule (II'b), le groupe R1-(C=O) est tel que défini précédemment et R4 représente un atome d'hydrogène.

12. Composition (C"1) telle que définie à l'une quelconque des revendications 9 ou 10 pour laquelle, dans les formules (I"a) et (I"b), ou bien s et p sont égaux à 0, ou bien s et p sont égaux à 1.

13. Formulation cosmétique à usage topique **caractérisée en ce qu'**elle comprend au moins un excipient cosmétiquement acceptable et une quantité efficace du composé de formule (I") telle que définie à l'une ou quelconque des revendications 7 à 9, ou de la composition (C"1) telle que définie à l'une quelconque des revendications 10 à 12.

14. Procédé non thérapeutique dans le but d'empêcher ou de ralentir l'apparition des signes du vieillissement de la peau humaine ou des lèvres ou bien d'éliminer lesdits signes, comprenant au moins une étape d'application sur la peau humaine ou les lèvres, d'une formulation cosmétique à usage topique comprenant au moins un excipient cosmétiquement acceptable et une quantité efficace d'au moins un composé de formule (I") telle que définie à l'une ou quelconque des revendications 7 à 9, ou de la composition (C"1) telle que définie à l'une quelconque des revendications 10 à 12.

## Patentansprüche

1. Nichttherapeutische Verwendung einer Verbindung der Formel (I'):
R'-O-CH₂-CH(OH)-[CH₂-O-CH₂]ₛ-[CH(OH)]ₚ-CH₂-O-R" (I'),
Formel (I'), in der s gleich 0 oder 1 ist und p gleich 0, 1, 2 oder 3 ist, R' und R", die identisch oder verschieden sind, entweder ein Wasserstoffatom oder einen einwertigen Rest der Formel (II'a) darstellen: 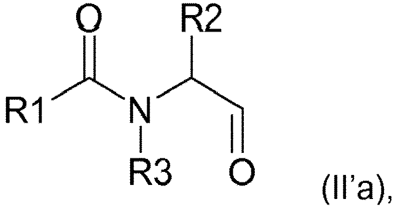 Formel (II'a), in der die Gruppe R1-C(=O)- einen Rest darstellt, der aus Octanoyl-, Decanoyl-, ω-Undecylenyl-, Dodecanoyl-, Tetradecanoyl-, Hexadecanoyl-, Octadecanoyl-, Cocoyl-, Eicosanoyl-, 9-Octadecenoyl-, Eicosenoyl-, 9,12-Octadecadienoyl- oder 9,12,15-Octadecatrienoyl-Resten ausgewählt ist, R2 ein Wasserstoffatom oder einen Rest darstellt, der aus Methyl-, Isopropyl-, Isobutyl-, 1-Methylpropyl-, Hydroxymethyl-, 1-Hydroxyethyl-, 4-Aminobutyl-, 3-Guanidinopropyl-, 3-Ureidopropyl-, (1-Aminocarbonyl-)methyl-, Carboxymethyl-, 2-Carboxyethyl-, 2- (Aminocarbonyl-)ethyl-, Benzyl-, 4-Hydroxybenzyl-, 3,4-Dihydroxybenzyl-, [1H-Indol-3-yl-]methyl-, (1H-Imidazol-4-yl-)methyl-, 3-Aminopropyl-Resten ausgewählt ist, und R3 ein Wasserstoffatom oder einen Methylrest darstellt; oder einen einwertigen Rest der Formel (II'b): 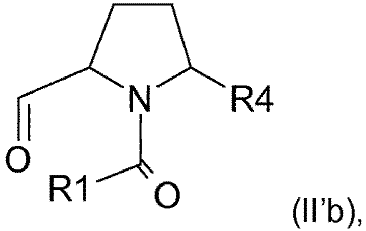 Formel (II'b), in der die Gruppe R₁-C(=O)- einen Rest darstellt, der aus Octanoyl-, Decanoyl-, ω-Undecylenoyl-, Dodecanoyl-, Tetradecanoyl-, Hexadecanoyl-, Octadecanoyl-, Cocoyl-, Eicosanoyl-, 9-Octadecenoyl-, Eicosenoyl-, 9,12-Octadecadienoyl- oder 9,12,15-Octadecatrienoyl-Resten ausgewählt ist und R4 ein Wasserstoffatom oder einen Hydroxyrest darstellt,
wobei:
- mindestens einer der Reste R' oder R" kein Wasserstoffatom darstellt,
- wenn keiner der Reste R' und R" ein Wasserstoffatom darstellt, R' und R" identisch sind, jedoch keinen einwertigen Rest der Formel (II'a) darstellen, in der die Gruppe R1-C(=O) den ω-Undecylenoylrest darstellt, R2 einen Benzylrest darstellt und R3 ein Wasserstoffatom darstellt und
- wenn einer der Reste R' und R" ein Wasserstoffatom darstellt, der andere keinen einwertigen Rest der Formel (II'a) darstellt, in der die Gruppe R1-C(=O) den ω-Undecylenoyl-Rest darstellt, R2 einen Benzylrest darstellt und R3 ein Wasserstoffatom darstellt,
wobei deren Verwendung dazu dient, das Auftreten von Alterserscheinungen der menschlichen Haut oder der Lippen zu verhindern oder zu verlangsamen oder diese Erscheinungen zu beseitigen.

2. Verwendung nach Anspruch 1 einer Zusammensetzung (C'1), umfassend für 100 % ihrer Masse:
- von 99 Gew.-% bis 20 Gew.-% mindestens einer Verbindung der Formel (I'a):
R'-O-CH₂-CH(OH)-[CH₂-O-CH₂]ₛ-[CH(OH)]ₚ-CH₂-O-H (I'a),
Formel (I'a) entsprechend der Formel (I') wie oben definiert, bei der R" ein Wasserstoffatom darstellt,
- von 1 Gew.-% bis 80 Gew.-% mindestens einer Verbindung der Formel (I'b):
R-O-CH₂-CH(OH)-[CH₂-O-CH₂]ₛ-[CH(OH)]ₚ-CH₂-0-R (Ib),
Formel (Ib) entsprechend der Formel (I') wie oben definiert, bei der R' und R" identisch sind und durch den Rest R dargestellt sind.

3. Verwendung nach einem der Ansprüche 1 oder 2, **dadurch gekennzeichnet, dass** in der Formel (II'a) R2 einen Rest darstellt, der aus Methyl-, Isopropyl-, Isobutyl-, 1-Methylpropyl- oder Benzyl-Resten ausgewählt ist, und dadurch, dass in der Formel (II'b) R4 ein Wasserstoffatom darstellt.

4. Verwendung nach einem der Ansprüche 1 bis 3, bei der in den Formeln (I'), (I'a) oder (I'b) entweder s und p gleich 0 oder s und p gleich 1 sind.

5. Nichttherapeutisches Verfahren zur Verhinderung oder Verlangsamung des Auftretens von Alterserscheinungen der menschlichen Haut oder der Lippen oder zur Beseitigung dieser Erscheinungen, das mindestens einen Schritt des Auftragens einer kosmetischen Formulierung zur topischen Anwendung auf die menschliche Haut oder die Lippen umfasst, die mindestens einen kosmetisch verträglichen Hilfsstoff und eine wirksame Menge der Verbindung der Formel (I') nach einem der Ansprüche 1 bis 4 umfasst.

6. Nichttherapeutisches Verfahren zur Verhinderung oder Verlangsamung des Auftretens von Alterserscheinungen der menschlichen Haut oder der Lippen oder zur Beseitigung dieser Erscheinungen, das mindestens einen Schritt des Auftragens einer kosmetischen Formulierung zur topischen Anwendung auf menschliche Haut oder Lippen umfasst, die mindestens einen kosmetisch verträglichen Hilfsstoff und eine wirksame Menge der Zusammensetzung (C'1) nach einem der Ansprüche 2 bis 4 umfasst.

7. Verbindung der Formel (I"), entsprechend der Formel (I') nach Anspruch 1, wobei jedoch gilt:
- wenn keiner der Reste R' und R" ein Wasserstoffatom darstellt und R' und R" identisch sind, stellen sie auch keinen einwertigen Rest der Formel (II'a) dar, in der die Gruppe R1-C(=O) den Tetradecanoylrest darstellt, R2 einen Benzylrest darstellt und R3 ein Wasserstoffatom darstellt und;
- wenn einer der Reste R' und R" ein Wasserstoffatom darstellt, stellt der andere auch keinen einwertigen Rest der Formel (II'a), in der die Gruppe R1-C(=O) den Tetradecanoylrest darstellt, R2 einen Benzylrest darstellt und R3 ein Wasserstoffatom darstellt, dar.

8. Verbindung der Formel (I") nach Anspruch 7, bei der:
- in der Definition des einwertigen Restes der Formel (II'a) die Gruppe R1-(C=O) und R3 wie oben definiert sind und R2 einen Rest darstellt, der aus den Methyl-, Isopropyl-, Isobutyl- und 1-Methylpropyl- oder Benzyl-Resten ausgewählt ist und
- in der Definition des einwertigen Restes der Formel (II'b) die Gruppe R1-(C=O) wie oben definiert ist und R4 ein Wasserstoffatom darstellt.

9. Verbindung der Formel (I") nach einem der Ansprüche 7 oder 8, in der entweder s und p gleich 0 oder s und p gleich 1 sind.

10. Zusammensetzung (C"1) umfassend für 100 % ihrer Masse:
- von 99 Gew.-% bis 20 Gew.-% mindestens einer Verbindung der Formel (I"a):
R'-O-CH₂-CH(OH)-[CH₂-O-CH₂]ₛ-CH(OH)]ₚ-CH₂-O-H (I"a),
Formel (I"a) entsprechend der Formel (I") nach Anspruch 7, bei der R" ein Wasserstoffatom darstellt,
- von 1 Gew.-% bis 80 Gew.-% mindestens einer Verbindung der Formel (I"b):
R-O-CH₂-CH(OH)-[CH₂-O-CH₂]ₛ-[CH(OH)]ₚCH₂-O-R (I"b),
Formel (I"b) entsprechend der Formel (I") nach Anspruch 7, in der R' und R" identisch sind und durch den Rest R dargestellt sind.

11. Zusammensetzung (C"1) nach Anspruch 10, bei der:
- in der Definition des einwertigen Restes der Formel (II'a) die Gruppe R1-(C=O) und R3 wie oben definiert sind und R2 einen Rest darstellt, der aus den Methyl-, Isopropyl-, Isobutyl- und 1-Methylpropyl- oder Benzyl-Resten ausgewählt ist und
- in der Definition des einwertigen Restes der Formel (II'b) die Gruppe R1-(C=O) wie oben definiert ist und R4 ein Wasserstoffatom darstellt.

12. Zusammensetzung (C"1) nach einem der Ansprüche 9 oder 10, bei der in den Formeln (I"a) und (I"b) entweder s und p gleich 0 oder s und p gleich 1 sind.

13. Kosmetische Formulierung zur topischen Anwendung, **dadurch gekennzeichnet, dass** sie mindestens einen kosmetisch verträglichen Hilfsstoff und eine wirksame Menge der Verbindung der Formel (I") nach einem der Ansprüche 7 bis 9 umfasst, oder der Zusammensetzung (C"1) nach einem der Ansprüche 10 bis 12umfasst.

14. Nichttherapeutisches Verfahren zur Verhinderung oder Verlangsamung des Auftretens von Alterserscheinungen der menschlichen Haut oder der Lippen oder zur Beseitigung dieser Erscheinungen, das mindestens einen Schritt des Auftragens einer kosmetischen Formulierung zur topischen Anwendung auf die menschliche Haut oder die Lippen umfasst, die mindestens einen kosmetisch verträglichen Hilfsstoff und eine wirksame Menge der Verbindung der Formel (I") nach einem der Ansprüche 7 bis 9 umfasst, oder der Zusammensetzung (C"1) nach einem der Ansprüche 10 bis 12 umfasst.

## Claims

1. Non-therapeutic use of a compound of formula (I'):
R'-O-CH₂-CH(OH)-[CH₂-O-CH₂]ₛ-[CH(OH)]ₚ-CH₂-O-R" (I'),
formula (I') in which s is equal to 0 or to 1, and p is equal to 0, 1, 2 or 3, R' and R", identical or different, represent either a hydrogen atom, or a monovalent radical of formula (II'a): 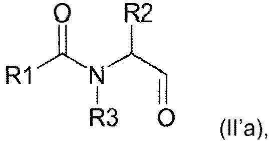 formula (II'a) in which the group R1-C(=O)- represents a radical chosen from among the octanoyl, decanoyl, ω-undecylenoyl, dodecanoyl, tetradecanoyl, hexadecanoyl, octadecanoyl, cocoyl, eicosanoyl, 9-octadecenoyl, eicosenoyl, 9,12-octadecadienoyl or 9,12,15-octadecatrienoyl radicals, R2 represent a hydrogen atom or a radical chosen from among the methyl, isopropyl, isobutyl, 1-methyl propyl, hydroxymethyl, 1-hydroxyethyl, 4-aminobutyl, 3-guanidino propyl, 3-ureido propyl, (1-amino carbonyl) methyl, carboxymethyl, 2-carboxyethyl, 2-(amino carbonyl) ethyl, benzyl, 4-hydroxy benzyl, 3,4-dihydroxy benzyl, [1H-indol-3-yl] methyl, (1H-imidazol-4-yl) methyl, 3-amino propyl radicals, and R3 represents a hydrogen atom or a methyl radical; or a monovalent radical of formula (II'b): 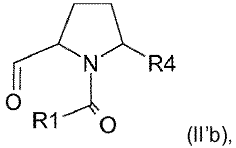 formula (II'b) in which the group R₁-C(=O)- represents a radical chosen from among the octanoyl, decanoyl, ω-undecylenoyl, dodecanoyl, tetradecanoyl, hexadecanoyl, octadecanoyl, cocoyl, eicosanoyl, 9-octadecenoyl, eicosanoyl, 9,12-octadecadienoyl or 9,12,15-octadecatrienoyl radicals and R4 represents a hydrogen atom or a hydroxy radical.
it being understood:
- that at least one of the radicals R' or R" does not represent a hydrogen atom,
- that when neither of the radicals R' and R" represents a hydrogen atom, R' and R" are identical but do not represent a monovalent radical of formula (II'a) in which the group R1-C(=O) represents the radical ω-undecylenoyl, R2 represents a benzyl radical and R3 represents a hydrogen atom, and
- that when one of the radicals R' and R" represents a hydrogen atom, the other does not represent a monovalent radical of formula (II'a) in which the group R1-C(=O) represents the radical w-undecylenoyl, R2 represents a benzyl radical and R3 represents a hydrogen atom,
said use being for the purpose of preventing or slowing the appearance of signs of ageing of the human skin or lips or even for eliminating said signs.

2. The use according to claim 1, of a composition (C'1) comprising for 100% of the mass thereof:
- from 99% by mass to 20% by mass of at least one compound of formula (I'a):
R'-O-CH₂-CH(OH)-[CH₂-O-CH₂]ₛ-[CH(OH)]ₚ-CH₂-O-H (I'a),
formula (I'a) corresponding to formula (I') as previously defined, in which R" represents a hydrogen atom,
- from 1% by mass to 80% by mass of at least one compound of formula (I'b):
R-O-CH₂-CH(OH)-[CH₂-O-CH₂]ₛ-[CH(OH)]ₚ-CH₂-O-R (Ib),
formula (Ib) corresponding to formula (I') as previously defined in which R' and R" are identical and represented by the radical R.

3. The use according to any of claims 1 or 2, **characterised in that** in the formula (II'a), R2 represents a radical chosen from among the methyl, isopropyl, isobutyl, 1-methyl propyl or benzyl radicals, and **in that** in formula (II'b), R4 represents a hydrogen atom.

4. The use according to any of claims 1 to 3 for which, in the formulas (I'), (I'a) or (I'b), either s and p are equal to 0 or s and p are equal to 1.

5. Non-therapeutic method for the purpose of preventing or slowing the appearance of signs of ageing of the human skin or lips or even for eliminating said signs, comprising at least a step of applying, on the human skin or lips, a cosmetic formulation for topical use comprising at least one cosmetically acceptable excipient and an effective quantity of the compound of formula (I'), according to any of claims 1 to 4.

6. Non-therapeutic method for the purpose of preventing or slowing the appearance of signs of ageing of the human skin or lips or even for eliminating said signs, comprising at least a step of applying, on the human skin or lips, a cosmetic formulation for topical use comprising at least one cosmetically acceptable excipient and an effective quantity of the composition (C'1), according to any of claims 2 to 4.

7. Compound of formula (I"), corresponding to formula (I') according to claim 1, but it being understood that:
- when neither of the radicals R' and R" represents a hydrogen atom and R' and R" are identical, they also do not represent a monovalent radical of formula (II'a) in which the group R1-C(=O) represents the radical tetradecanoyl, R2 represents a benzyl radical and R3 represents a hydrogen atom, and that;
- when one of the radicals R' and R" represents a hydrogen atom, the other also does not represent a monovalent radical of formula (II'a) in which the group R1-C(=O) represents the radical tetradecanoyl, R2 represents a benzyl radical and R3 represents a hydrogen atom.

8. Compound of formula (I") according to claim 7, for which:
- in the definition of the monovalent radical of formula (II'a), the group R1-(C=O) and R3 are as previously defined and R2 represents a radical chosen from among the methyl, isopropyl, isobutyl, 1-methyl propyl or benzyl radicals, and
- in the definition of the monovalent radical of formula (II'b), the group R1-(C=O) is as previously defined and R4 represents a hydrogen atom.

9. Compound of formula (I") according to any of claims 7 or 8, in which either s and p are equal to 0, or s and p are equal to 1.

10. Composition (C"1) comprising for 100% of its mass:
- from 99% by mass to 20% by mass of at least one compound of formula (I"a):
R'-O-CH₂-CH(OH)-[CH₂-O-CH₂]ₛ-[CH(OH)]ₚ-CH₂-O-H (I"a),
formula (I"a) corresponding to formula (I") according to claim 7, wherein R" represents a hydrogen atom,
- from 1% by mass to 80% by mass of at least one compound of formula (I"b):
R-O-CH₂-CH(OH)-[CH₂-O-CH₂]ₛ-[CH(OH)]ₚ-CH₂-O-R (I"b),
formula (I"b) corresponding to formula (I") according to claim 7, wherein R' and R" are identical and represented by the radical R.

11. Composition (C"1) according to claim 10, for which:
- in the definition of the monovalent radical of formula (II'a), the group R1-(C=O) and R3 are as previously defined and R2 represents a radical chosen from among the methyl, isopropyl, isobutyl, 1-methyl propyl or benzyl radicals, and
- in the definition of the monovalent radical of formula (II'b), the group R1-(C=O) is as previously defined and R4 represents a hydrogen atom.

12. Composition (C"1) according to any of claims 9 or 10 for which, in the formulas (I"a) and (I"b), either s and p are equal to 0 or s and p are equal to 1.

13. Cosmetic formulation for topical use **characterised in that** it comprises at least one cosmetically acceptable excipient and an effective quantity of the compound of formula (I") according to any of claims 7 to 9, or of composition (C"1) according to any of claims 10 to 12.

14. Non-therapeutic method for the purpose of preventing or slowing the appearance of signs of ageing of the human skin or lips or even for eliminating said signs, comprising at least one step of applying, on the human skin or lips, a cosmetic formulation for topical use comprising at least one cosmetically acceptable excipient and an effective quantity of at least one compound of formula (I") according to any one of claims 7 to 9, or of the composition (C"1) according to any of claims 10 to 12.
